Europäisches Patentamt

European Patent Office

Office européen des brevets

(11) Publication number: **0 245 796**
**A1**

# (12) EUROPEAN PATENT APPLICATION

(21) Application number: **87106712.0**

(22) Date of filing: **08.05.87**

(51) Int. Cl.³: **C 07 C 9/04**
**C 07 C 7/12**

(30) Priority: **16.05.86 US 864178**

(43) Date of publication of application:
**19.11.87 Bulletin 87/47**

(84) Designated Contracting States:
**BE DE FR GB IT NL**

(71) Applicant: **AIR PRODUCTS AND CHEMICALS, INC.**
**P.O. Box 538**
**Allentown, Pennsylvania 18105(US)**

(72) Inventor: **Sircar, Shivaji**
**1508 Bogie Avenue**
**Wescosville, PA 18106(US)**

(72) Inventor: **Koch, William Rohrer**
**R.D. 1, Box 1216**
**Fleetwood, PA 19522(US)**

(74) Representative: **Kador & Partner**
**Corneliusstrasse 15**
**D-8000 München 5(DE)**

(54) Recovery of methane from landfill gas.

(57) High purity methane and carbon dioxide are recovered from landfill gas in an integrated multi-column adsorption system having a temperature swing adsorption section (TSA) for pretreatment of the crude landfill gas to remove trace impurities therefrom, the thus cleaned gas being fed to a pressure swing adsorption section (PSA) for bulk separation of $CO_2$ from methane. Regeneration of the impurity-laden adsorbent bed of the TSA section is carried out using part of the $CO_2$ product gas recovered from PSA section which gas is heated to thermal regeneration temperature.

# RECOVERY OF METHANE FROM LAND FILL GAS

## TECHNICAL FIELD OF INVENTION

The present invention is concerned with the purification and recovery of pure methane from land fill gas (LFG).

## BACKGROUND OF THE INVENTION

Landfill gas contains a large number of trace hydrocarbon impurities and water which must be removed prior to the bulk separation of methane from carbon dioxide, which separation is generally carried out in a pressure swing adsorption (PSA) system. In order to obtain effective $CO_2/CH_4$ separation in the PSA system the water and hydrocarbon impurities need first to be removed from the crude LFG, since these impurities adversely effect the separation capacity of the selective adsorbent, such as zeolite, employed in the PSA system.

In a known previously utilized practice crude landfill gas was pretreated in a thermal swing adsorption (TSA) system to remove the water and hydrocarbon impurities prior to subjecting the LFG to separation of $CO_2/CH_4$ by PSA. The impurity-laden TSA adsorbent was regenerated, using part of the cleaned $CO_2$ and $CH_4$--containing stream or the eventual $CH_4$ product stream to heat the adsorbent. The same gas was used to cool the TSA adsorbent before a new cycle could be started. This procedure results in the loss of a significant amount of the cleaned feed gas ($CO_2 + CH_4$) or of the valuable separated product gas ($CH_4$).

## BRIEF SUMMARY OF THE INVENTION

In accordance with the present invention an integrated TSA-PSA process is employed wherein water and trace impurities present in the LFG are removed in the TSA pretreat section prior to bulk separation of components in the cleaned gas to obtain essentially pure $CH_4$ and $CO_2$ respectively. In the integrated TSA-PSA process of the invention the TSA

adsorbent beds are heated and cooled with a portion of the by-product $CO_2$ stream from the PSA section, which by-product stream is dry and free of impurities. Thus, there is no loss of valuable $CH_4$ in practice of the invention.

## BRIEF DESCRIPTION OF THE DRAWINGS

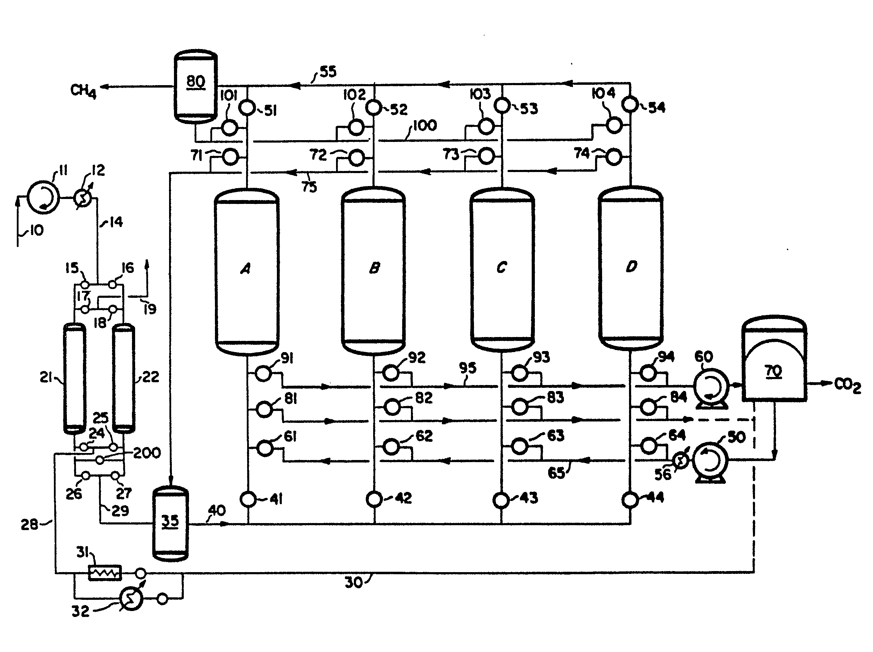

In the accompanying drawing the single Figure is a schematic flow diagram illustrative of an integrated TSA/PSA system for practice of the invention.

## DETAILED DESCRIPTION OF THE INVENTION

As shown in the drawing, the TSA pretreat section comprises two adsorbent columns 21, 22 operated alternately in parallel such that during the period that one of these columns is on the adsorption stroke receiving impure LFG, the impurity-laden adsorbent bed in the companion column is being subjected to regeneration. In addition to the valving system required for the sequence of operational steps employed in the pretreat section, other essential and conventional pieces of equipment in the pretreat section include a gas feed compressor 11 and aftercooler/condenser 12. The impurity-freed gas discharged from the pretreat section, comprised of about equal parts of $CH_4$ and $CO_2$, may be passed directly to one of the PSA columns, then on the adsorption stroke.

The PSA system illustrated comprises four adsorption Columns A, B, C, D, operated sequentially in parallel. In addition, the integrated system has among its major components a compressor 50 for $CO_2$, a vacuum pump 60, and a variable volume constant pressure or constant volume variable pressure storage vessel 70, in which part of the product gas is stored. The system further comprises vessels 35 and 80. Both of the vessels 35 and 80 are high pressure mixing vessels used as is hereinafter set out. The PSA section, as well as the TSA section are provided with the required switch valves and gas flow manifolds.

The TSA section, as such, is operated in conventional manner, the following sequence of steps being employed.

(a) Adsorption at super ambient pressure.

(b) Depressuring the adsorption column to ambient pressure level.

(c) Thermal regeneration of the adsorbent in the column at ambient pressure.

(d) Cooling the regenerated adsorbent in the column.

(e) Repressuring the cooled column to the adsorption pressure.

The cycle time of step (a) is equal to the sum of the cycle times for steps (b) to (e), thus allowing continuous flow of cleaned feed to the PSA section. A total cycle time of 4 to 16 hours can be used in the TSA section.

The PSA section has the following cyclic step sequence:

(1) Selective adsorption of $CO_2$ at super ambient pressure while discharging unadsorbed $CH_4$.

(2) Co-current $CO_2$ rinse at near feed pressure.

(3) Countercurrent depressure of the column to near ambient pressure.

(4) Countercurrent evacuation of the column to sub-atmospheric pressure level.

(5) Countercurrent repressurization of the column using part of the clean $CH_4$ product.

Assuming that column 21 has been brought to desired adsorption pressure (step e) for start of a TSA cycle, it is charged with LFG feed while column 22 is undergoing the regeneration sequence. The land fill off-gas typically contains 30-70% methane mixed with 70-30% $CO_2$ besides a large number of saturated and unsaturated hydrocarbons, aromatic hydrocarbons, chlorinated hydrocarbons, etc. as impurities in trace levels (ppm), as well as water. Such gas is delivered to the TSA system by line 10, and compressed by feed compressor 11 to a selected pressure level in the range of 30-200 psig and then cooled to near ambient temperature in the cooler-condensor 12 where any resulting condensate is removed. The compressed gas is then passed by line 14 through one of the TSA columns, in this instance 21, which had then been previously brought to feed pressure.

During the passage of the feed gas (step a) into and through column 21, valves 15 and 26 associated with that column are open while valves 17 and 24 are closed. Step (a) is continued for a predetermined time period during which the adsorbent in column 21 removes the trace

- 4 -

impurities and water, at which time the flow of feed into column 21 is discontinued by closing valves 15 and 26, and feed introduction switched to column 22 by opening valves 16 and 27.

The LFG, freed of impurities in column 21, or in column 22 in turn, is discharged through line 29 into high pressure mixing vessel 35. Alternatively the purified gas in line 29 may be sent directly to the PSA section, entering the column then on the adsorption stroke (1).

Regeneration of the impurity-laden bed in column 21 is carried out (steps b to e) during the time that column 22 is on the adsorption stroke. Column 21 is depressured (b) to ambient pressure level by opening valve 17 thereby effecting discharge of gas from that column into line 19 which may be vented to the atmosphere or sent to a flare.

When column 21 is at ambient pressure level, heat is supplied to the bed of adsorbent therein to drive off sorbed impurities (c).

The hot gas used for regeneration is part of the pure $CO_2$ separated from the $CH_4$ in the PSA section. The hot regeneration gas is supplied to the TSA section by line 28 through then opened valve 24 and discharged with desorbed products from column 21 into line 19 via open valve 17. At the end of the assigned heating period the bed in column 21 is cooled (d) by cold gas supplied through line 28 and passed through the bed via open valves 24 and 17 into discharge line 19.

At the termination of the cooling period, valve 17 is closed and column 21 is brought back to adsorption pressure by admission of compressed cleaned gas from companion column 22 or using part of the $CO_2$ product gas from the PSA section. In the latter modification the repressuring gas is admitted to column 21 via line 28 and open valve 24. If gas discharged from column 22 is to be used in repressuring column 21, valve 200 can be opened in a controlled fashion to connect vessels 21 and 22.

Table 1 below illustrates a time program for the TSA section employing a suggested four hour cycle, indicating valve positions during the cycle.

## TABLE 1

### VALVE POSITIONS IN TSA SECTION

| Column 21 | | Column 22 | | Valve | | | | | | | | |
|---|---|---|---|---|---|---|---|---|---|---|---|---|
| | | | | 15 | 16 | 17 | 18 | 24 | 25 | 26 | 27 | 200 |
| Ads. | (a) | Depr. | (b) | O | C | C | O | C | C | O | C | C |
| Ads. | (a) | Heat | (c) | O | C | C | O | C | O | O | C | C |
| Ads. | (a) | Cool | (d) | O | C | C | O | C | O | O | C | C |
| Ads. | (a) | Repr. | (e) | O | C | C | C | C | O/C | O | C | C/O |
| Depr. | (b) | Ads. | (a) | C | O | O | C | C | C | C | O | C |
| Heat | (c) | Ads. | (a) | C | O | O | C | O | C | C | O | C |
| Cool | (d) | Ads. | (a) | C | O | O | C | O | C | C | O | C |
| Repr. | (e) | Ads. | (a) | C | O | C | C | O/C | C | C | O | C/O |

O = Open     Ads. = Adsorption
C = Closed    Depr. = Depressurize
                Heat = Thermal regeneration
                Cool = Cooling bed
                Repr. = Repressurizing

During a four hour total cycle, each of the TSA columns alternately will be on the adsorption stroke for half that period (2 hours), then on steps (b) to (e) for two hours. Each of the steps (b) to (e) may be designed for equal 30 minute periods, or if preferred the two hours may be divided, assigning longer time periods for the heating and cooling steps (c) and (d) than that used in steps (b) and (e).

The hot gas employed in regeneration of the adsorbent beds of the TSA section is supplied by an effluent from the PSA section as hereinafter described. Such regeneration gas is passed by line 30 through a heater 31 into line 28, discharging into column 21 or 22 then on the thermal regeneration step (c), through open valve 24 or 25 respectively. At the termination of the heating step gas from line 30 is switched to flow through a cooler 32 before entering line 28, feeding the cooling gas to the column (21 or 22) then on step (d). The cooler may be eliminated if the column is cooled down to only ambient temperature. In that case cooling can be achieved by switching off the heater 31. The thermal regeneration may be carried out using gas heated to 250 to 900°F. In the subsequent cooling step the cooled gas is supplied to the column then on

- 6 -

step (d) at a temperature in the range of 40 to 120°F, to restore the column to a subambient or ambient temperature level.

The clean gas discharged from the TSA section through line 29 is passed into high pressure mixing vessel 35 from which it is withdrawn by line 40 and charged into one of the columns of the PSA section then on the adsorption stroke. Assuming that column A is on the adsorption stroke, having been previously brought to designed adsorption pressure, the feed gas comprised essentially of $CO_2$ and $CH_4$, is introduced into column A through then opened valve 41. The feed gas passes through the bed of adsorbent in column A whereby the $CO_2$ is selectively adsorbed and an effluent stream of substantially pure methane is discharged through open valve 51, into gas discharge manifold 55. During the adsorption stroke (1) the only valves 41 and 51 associated with column A are open. Adsorption is continued until the $CO_2$ level rises above the accepted level in the high purity $CH_4$ effluent.

At the termination of the adsorption stroke valves 41 and 51 are closed and feed gas flow is switched into one of the other columns of the PSA section. The adsorbent in column A being saturated with sorbed $CO_2$, is now rinsed at the prevailing super atmospheric pressure with a stream of high purity $CO_2$. The rinse stream is obtained from the storage vessel 70. Stored $CO_2$ is withdrawn from vessel 70 and compressed by compressor 50 to slightly above feed pressure level and cooled by cooling means 56 to near ambient temperature. The cooled $CO_2$ stream is passed into column A (step 2) through supply manifold 65, discharging into column A through open valve 61. The $CO_2$ rinse gas passes through column A in a direction co-current to that of the previous feed gas and is discharged from column A via open valve 71 into manifold 75.

The effluent from column A during the rinsing step (2) has a composition similar to that of the feed gas. It is mixed in mixer 35 with fresh purified feed gas from the TSA section and the mixture employed as feed to the PSA section adsorber columns. The rinsing step is continued until the column undergoing that step (2) is essentially saturated with pure $CO_2$.

At the termination of the rinsing of column A, valves 61 and 71 are closed and valve 81 is opened to depressure that column to near ambient pressure level (step 3) in a direction counter to that of the feed. At the reduced pressure the sorbed high purity $CO_2$ flows out of column A into discharge manifold 85. The gas in manifold 85 may be wholly or partly stored in vessel 70 (as indicated by the dashed line leading to that vessel) or it may be partly rejected. The gas, whether from vessel 70 or directly from line 85, may be used for regeneration of the TSA section as hereinbefore indicated. Since this gas is comprised of high purity $CO_2$ the non-used part may be collected in vessel 70 or withdrawn separately therefrom as a useful product for use or sale.

When column A is near the ambient pressure level valve 81 is closed and valve 91 is opened to initiate the evacuation step (4). Through open valve 91 the residual gas content (high purity $CO_2$) is withdrawn from column A by vacuum pump 60 via line 95 and may be passed into storage vessel 70. By using the vacuum pump 60 the column pressure is brought down to about 50-300 torr level by gas withdrawl in a direction counter to that of the feed. The part of the high purity $CO_2$ withdrawn by evacuation and not used in regeneration of the TSA section may be collected as useful product gas.

When desired evacuation has been completed, valve 91 is closed and valve 101 is opened for initiation of the repressuring step (5). The repressuring is effected by withdrawing part of the high purity $CH_4$ from vessel 80 via line 100 and flowing the same through open valve 101 into column A in a direction counter to feed until that column is brought back to approximately the feed pressure level. At the conclusion of the repressuring step (5) the described cycle of operations is repeated in the described sequence. Each of the PSA columns in turn undergoes the same sequence of steps as that described in connection with column A. Table 2 illustrates a time program for the various steps in the sequence of operations of the PSA section based on an embodiment employing a suggested 12 minute cycle and indicating the valve positions during the sequence. It will be understood, however, that the 12 minute cycle described is merely illustrative and that other time cycles may be employed in practice of the invention, with or without change in the number of adsorption columns utilized in the PSA section.

TABLE 6

**TIME**

| COL | 0 to 1.5 | 1.5 to 3.0 | 3.0 to 4.5 | 4.5 to 6.0 | 6.0 to 7.5 | 7.5 to 9.0 | 9.0 to 10.5 | 10.5 to 12.0 | | 0 to 1.5 | 1.5 to 3.0 | 3.0 to 4.5 | 4.5 to 6.0 | 6.0 to 7.5 | 7.5 to 9.0 | 9.0 to 10.5 | 10.5 to 12.0 |
|---|---|---|---|---|---|---|---|---|---|---|---|---|---|---|---|---|---|
| A | Ad | Ad | R | R | Dp | E | E | Pr | | Ad | Ad | R | R | Dp | E | E | Pr |
| B | E | Pr | Ad | Ad | R | R | Dp | E | | E | Pr | Ad | Ad | R | R | Dp | E |
| C | Dp | E | E | Pr | Ad | Ad | R | R | | Dp | E | E | Pr | Ad | Ad | R | R |
| D | R | R | Dp | E | E | Pr | Ad | Ad | | R | R | Dp | E | E | Pr | Ad | Ad |

**VALVE POSITION**

| | 0 to 1.5 | 1.5 to 3.0 | 3.0 to 4.5 | 4.5 to 6.0 | 6.0 to 7.5 | 7.5 to 9.0 | 9.0 to 10.5 | 10.5 to 12.0 | | | 0 to 1.5 | 1.5 to 3.0 | 3.0 to 4.5 | 4.5 to 6.0 | 6.0 to 7.5 | 7.5 to 9.0 | 9.0 to 10.5 | 10.5 to 12.0 |
|---|---|---|---|---|---|---|---|---|---|---|---|---|---|---|---|---|---|---|
| 41 | 0 | 0 | C | C | C | C | C | C | | 81 | C | C | C | C | 0 | C | C | C |
| 42 | C | C | 0 | 0 | C | C | C | C | | 82 | C | C | C | C | C | C | 0 | C |
| 43 | C | C | C | C | 0 | 0 | C | C | | 83 | 0 | C | C | C | C | C | C | C |
| 44 | C | C | C | C | C | C | 0 | 0 | | 84 | C | C | 0 | C | C | C | C | C |
| 51 | 0 | 0 | C | C | C | C | C | C | | 91 | C | C | C | C | C | 0 | 0 | C |
| 52 | C | C | 0 | 0 | C | C | C | C | | 92 | 0 | C | C | C | C | C | C | 0 |
| 53 | C | C | C | C | 0 | 0 | C | C | | 93 | C | 0 | 0 | C | C | C | C | C |
| 54 | C | C | C | C | C | C | 0 | 0 | | 94 | C | C | C | 0 | 0 | C | C | C |
| 61 | C | C | 0 | 0 | C | C | C | C | | 101 | C | C | C | C | C | C | C | C |
| 62 | C | C | C | C | 0 | 0 | C | C | | 102 | C | 0 | C | C | C | C | C | C |
| 63 | C | C | C | C | C | C | 0 | 0 | | 103 | C | C | C | 0 | C | C | C | C |
| 64 | 0 | 0 | C | C | C | C | C | C | | 104 | C | C | C | C | C | 0 | C | C |
| 71 | C. | C | 0 | 0 | C | C | C | C | | | | | | | | | | |
| 72 | C | C | C | C | 0 | 0 | C | C | | | | | | | | | | |
| 73 | C | C | C | C | C | C | 0 | 0 | | | | | | | | | | |
| 74 | 0 | 0 | C | C | C | C | C | C | | | | | | | | | | |

0 = Open
C = Closed

Ad: Adsorption
R: $CO_2$ Rinse
Dp: Depressuring
E: Evacuation
Pr: Pressuring

Table 2

- 8 -

0245796

6659C

The adsorbent employed in the pretreat section is one selective in retaining impurities present in the feed gas, which includes trace hydrocarbons heavier than methane, chlorinated hydrocarbons, etc., as well as residual water vapor. An example of the trace impurities is given in the following Table 3.

## TABLE 3

### Raw LFG Impurities

| Compound | Conc. (ppm) |
|---|---|
| Pentane | 5 |
| 1,1 dichloroethylene | 1 |
| dichloromethane | 12 |
| 1,2 dichloroethane | 4 |
| 1,1 dichloroethane | 8 |
| Hexane | 28 |
| Benzene | 23 |
| Iso-octane | 4 |
| Trichloroethane | 8 |
| Toluene | 210 |
| Tetrachloroethylene | 35 |
| 1,1,2 trichloroethylene | 0.1 |
| Chlorobenzene | 11 |
| Ethylbenzene | 54 |
| Xylenes | 116 |
| Nonane | 12 |
| Isopropyl benzene | 28 |
| Propyl benzene | 4 |
| Napthalene | 0.1 |

Typical adsorbent preferred for this purpose is activated carbon with which a water retaining adsorbent is admixed or provided as a separate layer in the TSA column. The water-retaining adsorbent component may be activated carbon, alumina, silica gel or an alumniosilicate molecular sieve zeolite.

For the separation of $CO_2/CH_4$ in the PSA section it is preferred to employ 13X zeolite as the $CH_4$-retaining adsorbent. Other adsorbents that may be employed in the PSA section include zeolites such as 5A, Silicalite, mordenite or activated carbon in lieu of the 13X zeolite or

- 10 -

in combination therewith. The zeolites (A, X, mordenite) can be ion exchanged with single ions from groups I and II metals or binary exchanged with two metals from these groups.

### EXAMPLE

The performance of the PSA section was evaluated in a bench scale unit after pretreatment to remove water and trace contaminants from the feed gas in a thermal swing adsorber consisting of 4.5 lbs. of an activated carbon and 1.5 lbs. of 13X zeolite. The impurities in the feed gas were those listed in Table 1. The pretreatment column was regenerated at about 850°F. The feed gas to the pretreatment column contained 42.5% $CO_2$ and 57.5% $CH_4$ and the pressure was 70 psig. An adsorption time of 4 hours was used. The pretreated feed was fed to at 70 psig and 21°C into a column containing 7 pounds of 13X zeolite. The unadsorbed effluent withdrawn from the column at 69 psig contained 99% $CH_4$. During the adsorption step 0.013 pound moles of feed gas was passed through the column. After termination of the adsorption step the column was rinsed with $CO_2$ of 99% purity at 72 psig and the rinse effluent was recycled as feed to the adsorption step. Following $CO_2$ rinsing the column was depressured to ambient pressure level and then evacuated to 95 torr. The obtained effluent comprised 98.5% $CO_2$, part of which was withdrawn as $CO_2$ product and the other part was recompressed as recycled $CO_2$ rinse. Following evacuation the column was repressured to 70 psig using part of the 99% pure $CH_4$ product and the cycle repeated.

The amounts of $CO_2$ products recovered from the system were 0.0074 pound moles $CH_4$ and .0056 pound moles of $CO_2$, each at 99% purity. Thus, the product recovery of both constituents of the feed mixture were 99% (within experimental error).

While the PSA system described and illustrated in the accompanying drawing employed a four adsorbent column configuration a five column configuration can be used to eliminate storage tank 80, or both storage tanks 70 and 80 can be eliminated by using a six adsorbent column configuration in the PSA section. Alternately a two or three adsorbent column system can be designed to accommodate the cycle described above by allowing the vacuum pump or the compressor to idle for parts of the cycle.

## What is Claimed:

1. In the process for purification and recovery of methane from landfill gas by subjecting such gas to pretreatment for removal of trace impurities therefrom followed by bulk separation of $CO_2$ from $CH_4$ from the pretreated gas by selective adsorption, the improvement which comprises:

    a. effecting such pretreatment in a multi-column temperature swing adsorption section integrated with a subsequent multi-column pressure swing adsorption section in which the separation of $CO_2$ from $CH_4$ is carried out,

    b. wherein a portion of the separated $CO_2$ product recovered from the pressure swing adsorption section is employed in regeneration of adsorbent in the temperature swing adsorption section,

    c. and wherein the time cycle of adsorption and regeneration in said temperature swing adsorption section is independent of that employed in said pressure swing adsorption section.

2. The improvement as defined in Claim 1 wherein said bulk separation effected in said pressure swing adsorption section comprises the sequence of steps: (1) selective adsorption of $CO_2$ at super ambient pressure during a predetermined time period while discharging unadsorbed $CH_4$ effluent, (2) co-current rinsing of the $CO_2/CH_4$-laden column with $CO_2$ product gas at near feed pressure, (3) countercurrent depressuring of the rinsed column to near ambient pressure followed by, (4) evacuation of said column to sub-atmospheric pressure level thereby removing sorbed $CO_2$ from said column, and (5) restoring the column to super ambient pressure by countercurrent introduction therein of part of the $CH_4$ produced in step (1).

3. The improvement as defined in Claim 2 wherein the pretreatment of the landfill gas comprises the sequence of step: (a) selective adsorption of contained impurities therefrom in an adsorbent bed at super ambient pressure during a predetermined time period while discharging an unadsorbent effluent consisting essentially of $CO_2$ and $CH_4$, (b) there-

after depressuring said bed to ambient pressure level and at said ambient pressure level, (c) thermally regenerating the impurity-laden bed with hot gas obtained by heating a portion of the $CO_2$ product gas from said pressure swing adsorption section, following by (d) cooling the bed and (e) restoring the bed to super ambient adsorption pressure.

4. The improvement as defined in Claim 3 wherein the total time for carrying out steps (b) through (e) is equal to the cycle time for step (a).

5. The improvement as defined in Claim 4 wherein the total cycle time for steps (a) through (e) is in the range of 4 to 16 hours.

6. The improvement as defined in Claim 2 wherein the total cycle time for steps 1 to 5 in said pressure swing cycle is 2 to 60 minutes.

7. The improvement as defined in Claim 6 wherein the pressure swing during the cycle in the bulk separation of $CO_2$ from $CH_4$ ranges from sub-atmospheric level at 50-200 torr during evacuation of the column to super ambient pressure in the range of 50-200 psig during the adsorption step.

8. The improvement as defined in Claim 3 wherein the thermal regeneration of the impurity-laden adsorbent in step (c) is effected at a temperature in the range of 250 to 900°F.

9. The improvement as defined in Claim 3 wherein following (c) regeneration and (d) cooling, the bed is restored to super ambient pressure level by introduction therein of part of the cleaned gas withdrawn from a companion column of the temperature swing adsorption section.

10. The improvement as defined in Claim 3 wherein following (c) regeneration and (d) cooling, the bed is restored to super ambient pressure level by introduction therein of part of the pure $CO_2$ product gas from the pressure swing adsorption section.

**European Patent Office**

**EUROPEAN SEARCH REPORT**

Application number

EP 87 10 6712

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| P,X | EP-A-0 193 716 (AIR PRODUCTS AND CHEMICALS) * Pages 3-4,7-8,10 * --- | 1,2,6, 7 | C 07 C 9/04 C 07 C 7/12 |
| A | EP-A-0 008 882 (AIR PRODUCTS AND CHEMICALS) --- | | |
| A | GB-A-2 000 047 (CALGON CORP.) ----- | | |

| | TECHNICAL FIELDS SEARCHED (Int Cl 4) |
|---|---|
| | C 07 C 9/00 C 07 C 7/00 |

The present search report has been drawn up for all claims

| Place of search | Date of completion of the search | Examiner |
|---|---|---|
| THE HAGUE | 29-07-1987 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82